(19) Europäisches Patentamt / European Patent Office / Office européen des brevets

(11) **EP 4 445 755 A1**

(12) **EUROPEAN PATENT APPLICATION**
published in accordance with Art. 153(4) EPC

(43) Date of publication:
**16.10.2024 Bulletin 2024/42**

(21) Application number: **21967156.7**

(22) Date of filing: **08.12.2021**

(51) International Patent Classification (IPC):
**A24D 1/20** $^{(2020.01)}$       **A24F 40/20** $^{(2020.01)}$

(52) Cooperative Patent Classification (CPC):
**A24F 40/20; A24D 1/027; A24D 1/045; A24D 1/20; A24D 3/17; A24F 40/40; A24F 40/46; A24F 47/00; A61M 11/042; A61M 15/06**

(86) International application number:
**PCT/JP2021/045032**

(87) International publication number:
**WO 2023/105657 (15.06.2023 Gazette 2023/24)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Japan Tobacco Inc.**
**Tokyo 105-6927 (JP)**

(72) Inventors:
• **KATAYAMA, Kazuhiko**
**Tokyo 130-8603 (JP)**
• **UCHII, Kimitaka**
**Tokyo 130-8603 (JP)**
• **MOTODAMARI, Tetsuya**
**Tokyo 130-8603 (JP)**
• **HARUKI, Keisuke**
**Tokyo 130-8603 (JP)**

(74) Representative: **Hoffmann Eitle**
**Patent- und Rechtsanwälte PartmbB**
**Arabellastraße 30**
**81925 München (DE)**

(54)    **NON-COMBUSTION-HEATING-TYPE STICK, AND INHALATION SYSTEM**

(57)    This non-combustion-heating-type stick 2 comprises a substrate part 10 including an aerosol source, a tubular cooling part 20 for cooling vapor generated due to the substrate part 10 being heated to generate an aerosol, and a filter part 30 through which the aerosol passes. An open hole V through which air is channeled from the exterior to the interior is formed in the cooling part 20 at a position that is at least 7 mm from the boundary between the cooling part 20 and the filter part 30, or that is within 13 mm from the boundary between the cooling part and the substrate part.

FIG. 2

FIRST SIDE ← CENTERLINE DIRECTION → SECOND SIDE

EP 4 445 755 A1

## Description

Technical Field

The present invention relates to a non-combustion-heating-type stick and an inhalation system.

Background Art

For example, a smoking article described in PTL 1 includes a body of smokable material and a filter assembly. The filter assembly includes a cooling segment, a filter segment adjacent to the cooling segment, and a mouth end segment to be received in a mouth of a user. The smoking article is configured such that, when the smokable material is fully inserted into an apparatus, a first section of the cooling segment is within the apparatus and a second section of the cooling segment extends outside of the apparatus. The second section of the cooling segment includes a ventilation region that enables air to flow into the cooling segment to mix with at least one volatilized component of the smokable material.

A heat-not-burn smoking article described in PTL 2 includes: a tobacco-containing segment containing tobacco and an aerosol former; a tubular cooling segment having a perforation on the outer circumferential surface thereof; and a tubular member having an inside diameter smaller than that of the cooling segment, wherein a mouth-side end of the cooling segment is joined to the member, and wherein the perforation is provided at a position 2 to 4 mm from the joint face.

Citation List

Patent Literature

PTL 1: Japanese Unexamined Patent Application Publication (Translation of PCT Application) No. 2019-518450
PTL 2: International Publication No. WO2020-100927

Summary of Invention

Technical Problem

In an inhaler device in which a substrate including an aerosol source is heated to generate an aerosol, it is preferable that finite energy that can be supplied for heating is utilized to increase the amount of the aerosol that can be supplied to the oral cavity of the user.

It is an object of the present invention to provide a non-combustion-heating-type stick etc. in which the amount of an aerosol that can be supplied to the oral cavity can be increased.

Solution to Problem

A first feature of the invention completed for achieving the foregoing object is a non-combustion-heating-type stick including: a substrate including an aerosol source; a tubular cooling section for cooling vapor generated by heating the substrate to thereby generate an aerosol; and a filter section through which the aerosol passes, wherein a perforation that allows air to flow from an outside of the cooling section into an inside of the cooling section is formed in the cooling section at a position at least 7 mm from a boundary between the cooling section and the filter section or at a position within 13 mm from a boundary between the cooling section and the substrate.

In a second feature, the perforation may be formed at a position at least 8 mm from the boundary between the cooling section and the substrate.

In a third feature, the perforation may be formed at a position within 12 mm from the boundary between the cooling section and the filter section.

From a different point of view, a fourth feature is an inhalation system including: a non-combustion-heating-type stick including a substrate including an aerosol source, a tubular cooling section for cooling vapor generated by heating the substrate to thereby generate an aerosol, and a filter section through which the aerosol passes; and an inhaler device including a holder that holds the non-combustion-heating-type stick, a heater that heats the substrate, and a housing that accommodates the holder and the heater, wherein, in the inhaler device, the holder holds at least the substrate of the non-combustion-heating-type stick inserted into the housing through an opening formed in the housing with at least part of the filter section exposed to an outside of the housing, and wherein, in the cooling section of the non-combustion-heating-type stick, a perforation that allows air to flow from an outside of the cooling section into an inside of the cooling section is formed at a position that, with the non-combustion-heating-type stick held by the holder, is located within the housing.

In a fifth feature, in the holder with the non-combustion-heating-type stick held thereby, a gap may be formed between an inner surface of the holder and an outer surface of a portion of the cooling section of the non-combustion-heating-type stick, which portion has the perforation formed therein.

In a sixth feature, the holder may include an insertion section disposed on an opening side and a compression section that is disposed on an opposite side from the opening with respect to the insertion section and that compresses the non-combustion-heating-type stick in a direction intersecting an insertion direction, and the insertion section may have a cylindrical shape having an inside diameter larger than an outside diameter of the outer surface of the non-combustion-heating-type stick. With the holder holding the non-combustion-heating-type stick, the perforation may be located within the insertion section.

In a seventh feature, the perforation may be formed at a position at least 7 mm from a boundary between the cooling section and the filter section or at a position within 13 mm from a boundary between the cooling section and the substrate. Advantageous Effects of Invention

According to the first feature, the amount of the aerosol that can be supplied to the oral cavity can be increased.

In the second feature, the amount of air heated by heating the substrate and flowing through the perforation into the cooling section can be smaller than that when, for example, the perforation is formed at a position less than 8 mm from the boundary between the cooling section and the substrate. Therefore, the amount of the aerosol that can be supplied to the oral cavity of the user can be increased.

In the third feature, the perforation is unlikely to be closer to a portion for heating the substrate than when, for example, the perforation is formed at a position more than 12 mm from the boundary between the cooling section and the filter section. Therefore, the amount of the aerosol that can be supplied to the oral cavity of the user can be increased.

In the fourth feature, the perforation can be easily located closer to the substrate with respect to the boundary between the cooling section and the filter section. Therefore, the amount of the aerosol that can be supplied to the oral cavity can be increased.

According to the fifth feature, air can easily flow into the cooling section through the perforation.

In the sixth feature, with the holder holding the stick, the gap is formed with high precision between the inner surface of the insertion section and the outer surface of the portion of the cooling section, which portion has the perforation formed therein. Therefore, air can be easily flow into the cooling section through the perforation.

In the seventh feature, the inhaler device in one of the fourth to sixth features is used for inhalation, and therefore the amount of the aerosol that can be supplied to the oral cavity can be increased.

Brief Description of Drawings

[Fig. 1] An illustration schematically showing an example of the schematic structure of an inhalation system according to a first embodiment.
[Fig. 2] An illustration showing an example of a vertical cross section of the stick according to the first embodiment.
[Fig. 3] An illustration showing an example of the schematic structure of a holder, the illustration being an enlarged view of portion III in Fig. 1.
[Fig. 4] An illustration showing an example of a cross section along IV-IV in Fig. 3.
[Figs. 5A and 5B] 5A: An illustration showing an example of a cross section along Va-Va in Fig. 4. 5B: An illustration showing an example of a cross section along Vb-Vb in Fig. 4.
[Fig. 6] An illustration showing an example of the flow of air during inhalation in the inhalation system.
[Fig. 7] An illustration showing a vertical cross section of a stick according to a second embodiment.

Description of Embodiments

Embodiments of the present invention will next be described in detail with reference to the accompanying drawings. In the drawings, the same parts are designated by the same numerals.

<First embodiment>

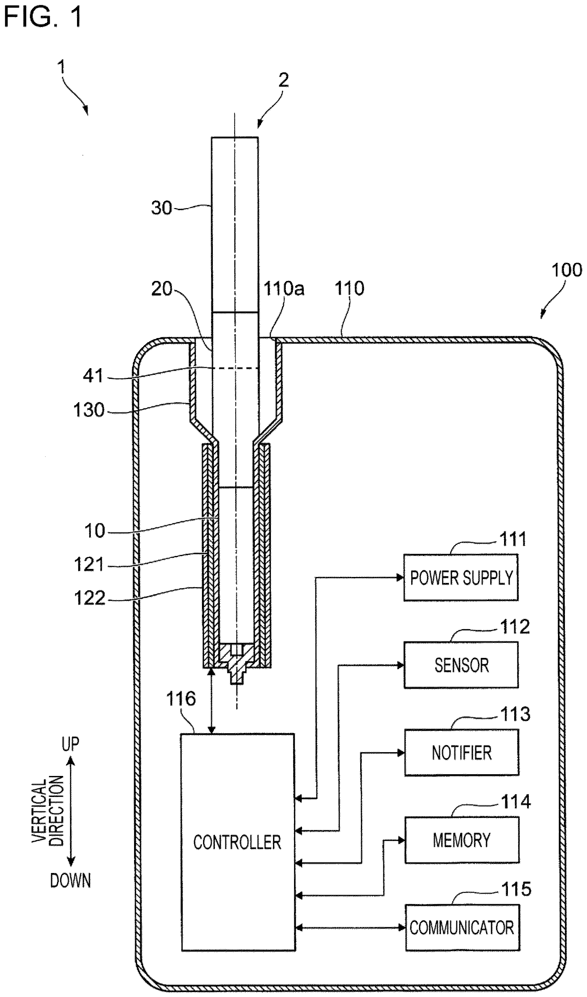

Fig. 1 is an illustration schematically showing an example of the schematic structure of an inhalation system 1 according to a first embodiment.

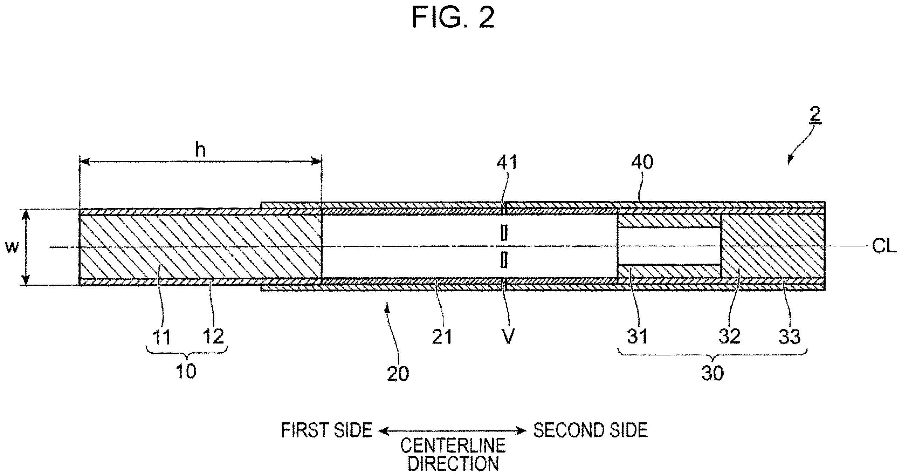

Fig. 2 is an illustration showing an example of a vertical cross section of a stick 2 in the first embodiment.

The inhalation system 1 includes: a non-combustion-heating-type stick (which may be hereinafter referred to also as a "stick") 2 including an aerosol source that generates an aerosol when heated; and an inhaler device 100 that heats the stick 2 to generate the aerosol.

(Stick 2)

The stick 2 includes a substrate 10, a cooling section 20, and a filter section 30. The substrate 10 is formed into a cylindrical shape. Hereinafter, the direction of the centerline CL of the substrate 10 may be referred to as a "centerline direction." The stick 2 further includes a tipping paper 40 wrapped around the substrate 10, the cooling section 20, and the filter section 30 arranged in this order in the centerline direction to integrate them. One end side in the centerline direction (the left side in Fig. 2) may be referred to as a first side, and the other end side in the centerline direction (the right side in Fig. 2) may be referred to as a "second side." The first side is the end side to be inserted into the inhaler device 100. The second side is the opposite side from the first side and is the end side that is to be held in the user's mouth for inhalation. A cross section along the centerline direction is referred to as a "vertical cross section," and a cross section cut along a plane orthogonal to the centerline direction is defined as a "transverse cross section."

[Substrate 10]

The substrate 10 includes an aerosol source 11 that, when heated, generates a vapor that is to form an aerosol and a wrapping paper 12 that covers the outer circumferential surface of the aerosol source 11. The substrate 10 is formed into a cylindrical shape with the wrapping paper 12 wrapped around the aerosol source 11. The aerosol source 11 may be, for example, a material derived from tobacco such as shredded tobacco or a processed material produced by processing a tobacco raw material into a granular, sheet, or powder form. The aerosol source 11 may contain a material not derived from tobacco and produced from a plant (such as mint or herb) other than tobacco. For example, the aerosol source 11 may contain a flavoring agent. No particular limitation is imposed on the type of flavoring agent. From the viewpoint of imparting a good flavor, the flavoring agent is particularly preferably menthol. One flavoring agent may be used alone, or two or more may be used in combination. When the inhaler device 100 is a medical inhaler, the aerosol source 11 may contain a medicine to be inhaled by a patient. The aerosol source 11 is not limited to a solid and may be a liquid such as water or polyhydric alcohol such as glycerin or propylene glycol. The substrate 10 is to be accommodated in the internal space of a holder 130 with the stick 2 held by the holder 130.

Preferably, the substrate 10 including the aerosol source 11 wrapped with the wrapping paper 12 has a cylindrical shape in which the aspect ratio defined by the following formula (1) is 1 or more.

$$\text{Aspect ratio} = h / w \qquad (1)$$

w is the width of the transverse cross section of the substrate 10, and h is the dimension of the substrate 10 in the centerline direction. It is preferable that $h \geq w$. No particular limitation is imposed on the shape of the transverse cross section, and the shape of the transverse cross section may be polygonal, rounded polygonal, circular, elliptical, etc. The width w is the diameter when the transverse cross section is circular, the major axis when the transverse cross section is elliptical, and the diameter of the circumscribed circle or the major axis of the circumscribed ellipse when the transverse cross section is polygonal or rounded polygonal. The width of the transverse cross section of the aerosol source 11 included in the substrate 10 is preferably 4 mm or more and 9 mm or less.

The dimension h of the substrate 10 in the centerline direction may be appropriately changed according to the size of the product but is generally 10 mm or more, preferably 12 mm or more, more preferably 15 mm or more, and still more preferably 18 mm or more. The dimension h of the substrate 10 in the centerline direction is generally 70 mm or less, preferably 50 mm or less, more preferably 30 mm or less, and still more preferably 25 mm or less.

No particular limitation is imposed on the ratio of the dimension h of the substrate 10 to the dimension of the stick 2 in the centerline direction. From the viewpoint of the balance between the delivery amount of the aerosol and the temperature of the aerosol, the ratio is generally 10% or more, preferably 20% or more, more preferably 25% or more, and still more preferably 30% or more. The ratio of the dimension h of the substrate 10 to the dimension of the stick 2 is generally 80% or less, preferably 70% or less, more preferably 60% or less, still more preferably 50% or less, particularly preferably 45% or less, and most preferably 40% or less.

No particular limitation is imposed on the content of the aerosol source 11 in the substrate 10, but the content may be 200 mg or more and 800 mg or less and is preferably 250 mg or more and 600 mg or less. The above range is particularly suitable when the substrate 10 has a circumferential length of 22 mm and a dimension in the centerline direction of 20 mm.

A description will be given of the aerosol source 11 containing shredded tobacco. No particular limitation is imposed on the material of the shredded tobacco contained in the aerosol source 11, and any well-known material such as lamina or midrib may be used. The material may be prepared by grinding dried tobacco leaves to an average grain diameter of 20 $\mu$m or more and 200 $\mu$m or less to obtain ground tobacco, then making the ground tobacco uniform, forming the resulting ground tobacco into a sheet (hereinafter referred to simply as a uniform sheet), and then shredding the sheet. Alternatively, a so-called strand type may be used. Specifically, a uniform sheet having a dimension equivalent to the dimension of the substrate 10 in the centerline direction is shredded substantially horizontally in the centerline direction of the substrate 10, and the shredded product is filled into the aerosol source 11.

The width of the shredded tobacco is preferably 0.5 mm or more and 2.0 mm or less because it is to be filled into the aerosol source 11.

As for the tobacco leaves used to produce the shredded tobacco and the uniform sheet, various types of tobacco may be used. Examples of the type of tobacco include a flue cured type, a burley type, an oriental type, native species, other Nicotiana tabacum varieties, Nicotiana rustica varieties, and mixtures thereof. As for the mixtures, different types may be appropriately blended so as to obtain an intended taste. The details of the varieties of tobacco are disclosed in "Tobacco no Jiten (Dictionary of Tobacco), Tobacco Academic Studies Center, March 31, 2009." There are a plurality of conventional methods for producing a uniform sheet, i.e., methods for shredding tobacco leaves and forming the shredded leaves into a uniform sheet. A first example is a method for producing a sheet made through a paper making process. A second example is a method including mixing the shredded tobacco leaves and a suitable solvent such as water uniformly to obtain a uniform mixture, casting the uniform mixture thinly onto a metallic sheet or a metallic sheet belt, and drying the mixture to produce a cast sheet. A third example is a method including mixing the shredded tobacco leaves and a suitable solvent such as water uniformly to obtain a uniform mixture and extruding the mixture into a sheet shape to produce a rolled sheet. The details of the types of uniform sheets are disclosed in "Tobacco no Jiten (Dictionary of Tobacco), Tobacco Academic Studies Center, March 31, 2009."

The content of moisture in the aerosol source 11 with respect to the total mass of the aerosol source 11 is, for example, 10% by mass or more and 15% by mass or less and preferably 11% by mass or more and 13% by mass or less. When the moisture content is as described above, the occurrence of stains on the wrapping paper is prevented, and the machinability during production of the substrate 10 is improved.

No particular limitation is imposed on the aerosol source 11, and the aerosol source 11 may contain extracts from various natural products and/or constituent components thereof according to the intended application. Examples of the extracts and/or the constituent components thereof include glycerin, propylene glycol, triacetin, 1,3-butanediol, and mixtures thereof.

No particular limitation is imposed on the content of the extracts and/or the constituent components thereof in the aerosol source 11. From the viewpoint of generating the aerosol sufficiently and imparting a good flavor, the content with respect to the total mass of the aerosol source 11 is generally 5% by mass or more and preferably 10% by mass or more. The content of the extracts and/or the constituent components thereof in the aerosol source 11 is generally 50% by mass or less and preferably 15% by mass or more and 25% by mass or less.

No particular limitation is imposed on the filling density in the aerosol source 11. From the viewpoint of ensuring the performance of the stick 2 and imparting a good flavor, the filling density is generally 250 mg/cm$^3$ or more and preferably 300 mg/cm$^3$ or more. The filling density in the aerosol source 11 is generally 400 mg/cm$^3$ or less and preferably 350 mg/cm$^3$ or less.

The aerosol source 11 may be formed of a tobacco sheet. The number of tobacco sheets may be one or may be two or more.

In one exemplary mode of the aerosol source 11 formed of one tobacco sheet, the tobacco sheet having a side length equivalent to the centerline direction dimension of a body to be filled is folded a plurality of times parallel to the centerline direction of the to-be-filled body (to form a so-called gathered sheet) and then filled into the to-be-filled body. In another mode, the tobacco sheet with a side length equivalent to the centerline direction dimension of the to-be-filled body is rolled up in a direction orthogonal to the centerline direction of the to-be-filled body and then filled into the to-be-filled body.

In one exemplary mode of the aerosol source 11 formed of two or more tobacco sheets, the tobacco sheets each having a side length equivalent to the centerline direction dimension of the to-be filled body are rolled up in a direction orthogonal to the centerline direction of the to-be-filled body such that the tobacco sheets are disposed concentrically, and the rolled tobacco sheets are filled into the to-be-filled body. The phrase "disposed concentrically" means that the tobacco sheets are disposed such that the centers of all the tobacco sheets are located at substantially the same position.

The two or more tobacco sheets may all have the same composition or the same physical properties, or some or all of the tobacco sheets may have different compositions or physical properties. The thicknesses of the tobacco sheets may be the same or different.

No particular limitation is imposed on the thickness of each tobacco sheet. However, from the viewpoint of the tradeoff between heat transfer efficiency and strength, the thickness is preferably 150 μm or more and 1000 μm or less and more preferably 200 μm or more and 600 μm or less.

The aerosol source 11 may be produced by preparing a plurality of tobacco sheets having different widths, laminating the tobacco sheets to prepare a laminate such that its width decreases from the first side to the second side, causing the laminate to pass through a rolling tube to thereby roll up the laminate.

With this production method, the plurality of tobacco sheets extend in the centerline direction and are disposed concentrically with the CL as the center

In this production method, it is preferable that the laminate is prepared such that a non-contact portion is formed between adjacent rolled tobacco sheets. When the non-contact portion (gap) is present between tobacco sheets such that the tobacco sheets are not in contact with each other, a flavor flow path is maintained, and the delivery efficiency of the flavor component can be increased. On the other hand, since heat from a heater 121 can be transferred to outer tobacco sheets through contact portions of the plurality of tobacco sheets, high heat transfer efficiency can be maintained.

Examples of a method for preparing the laminate in which non-contact portions in which tobacco sheets are not in contact with each other are provided between the tobacco sheets include a method in which embossed tobacco sheets are used, a method in which adjacent tobacco sheets are laminated without bonding their entire surfaces together, a method in which adjacent tobacco sheets are laminated with parts thereof bonded together, and a method in which adjacent laminated tobacco sheets are partially or entirely bonded together weakly such that they can be separated after the rolling-up process.

When the substrate 10 including the wrapping paper 12 is prepared, the wrapping paper 12 may be disposed on the first-side end face of the laminate.

No particular limitation is imposed on the filling density in the aerosol source 11. From the viewpoint of ensuring the performance of the stick 2 and imparting a good flavor, the filling density is generally 250 mg/cm$^3$ or more and preferably 300 mg/cm$^3$ or more. The filling density in the aerosol source 11 is generally 400 mg/cm$^3$ or less and preferably 350 mg/cm$^3$ or less.

A polyol such as glycerin, propylene glycol, or 1,3-butanediol may be added to the tobacco sheet. The amount of the polyol added to the tobacco sheet with respect to the dry mass of the tobacco sheet is preferably 5% by mass or more and 50% by mass or less and more preferably 15% by mass or more and 25% by mass or less.

The tobacco sheet can be appropriately produced using a well-known method such as a paper making method, a slurry method, or a rolling method. The uniform sheet described above may also be used.

When the paper making method is used, a method including the following steps can be used for the production. 1) Dried tobacco leaves are coarsely ground and extracted with water to separate the leaves into a water extract and a residue. 2) The water extract is dried under reduced pressure and concentrated. 3) Pulp is added to the residue. The mixture is formed into fibers using a refiner, and the fibers are subjected to paper making to produce a sheet. 4) The concentrate of the water extract is added to the produced sheet, and the sheet is dried to obtain a tobacco sheet. In this case, the step of removing some components such as nitrosamines may be added (see Japanese Unexamined Patent Application Publication (Translation of PCT Application) No. 2004-510422).

When the slurry method is used, a method including the following steps can be used for the production. 1) Water, pulp, a binder, and ground tobacco leaves are mixed. 2) The mixture is thinly spread (cast) and dried. In this case, the step of irradiating the slurry prepared by mixing water, the pulp, the binder, and the ground tobacco leaves with ultraviolet rays or X-rays to remove some components such as nitrosamines may be added.

Moreover, a nonwoven fabric-like tobacco sheet produced by a method including the following steps may be used, as described in International Publication No. WO2014/104078. 1) Granular tobacco leaves and a binder are mixed. 2) The mixture is sandwiched between nonwoven fabrics. 3) The laminate is formed into a given shape by heat fusion to thereby obtain a nonwoven fabric-like tobacco sheet.

The type of raw material tobacco leaves used in the above methods may be the same as that described for the aerosol source 11 containing shredded tobacco.

No particular limitation is imposed on the composition of the tobacco sheet. For example, the content of the tobacco raw material (tobacco leaves) with respect to the total mass of the tobacco sheet is preferably 50% by mass or more and 95% by mass or less. The tobacco sheet may contain a binder, and examples of the binder include guar gum, xanthan gum, carboxymethyl cellulose, and a sodium salt of carboxymethyl cellulose. The amount of the binder with respect to the total mass of the tobacco sheet is preferably 1% by mass or more and 10% by mass or less. The tobacco sheet may further contain an additional additive. Examples of the additive include fillers such as pulp.

No particular limitation is imposed on the structure of the wrapping paper 12 used for the substrate 10, and a general structure may be used. For example, a wrapping paper containing pulp as a main component may be used. The pulp may be made of wood pulp such as soft wood pulp or hard wood pulp or may be produced using a pulp mixture containing non-wood pulp generally used for the wrapping paper 12 for tobacco products such as flax pulp, hemp pulp, sisal pulp, or esparto.

Examples of the type of pulp that can be used include chemical pulp, ground pulp, chemiground pulp, and thermomechanical pulp that are produced, for example, by a kraft cooking method, an acidic/neutral/alkaline sulfite cooking method, or a soda salt cooking method.

In a paper making step using pulp in, for example, a fourdrinier machine, a cylinder paper machine, or a cylinder-tanmo complex paper machine, the texture of the pulp is controlled to distribute the pulp uniformly to thereby produce the wrapping paper 12. If necessary, a wet strength agent may be added to impart water resistance to the wrapping paper 12, or a sizing agent may be added to adjust the quality of printing on the wrapping paper 12. Moreover, internal auxiliary agents for paper making such as aluminum sulfate, an anionic, cationic, nonionic, or amphoteric retention aid, a freeness improving agent, and a strength agent may be added, and additives for paper making such as a dye, a pH modifier, an antifoaming agent, a pitch control agent, and a slime control agent may also be added.

The basis weight of the base sheet of the wrapping paper 12 is, for example, generally 20 gsm or more and preferably 25 gsm or more. The basis weight is generally 65 gsm or less, preferably 50 gsm or less, and still more preferably 45 gsm or less.

No particular limitation is imposed on the thickness of the wrapping paper 12. From the viewpoint of stiffness, air permeability, and the ease of adjustment during paper making, the thickness is generally 10 $\mu$m or more, preferably 20 $\mu$m or more, and more preferably 30 $\mu$m or more. The thickness of the wrapping paper 12 is generally 100 $\mu$m or less, preferably 75 $\mu$m or less, and still more preferably 50 $\mu$m or less.

The wrapping paper 12 for producing the substrate 10 may have a square shape or a rectangular shape.

When the wrapping paper 12 is used to wrap the aerosol source 11, one side length of the wrapping paper 12 is, for example, about 12 mm or more and about 70 mm or less, and the other side length is, for example, 15 mm or more and 28 mm or less. The other side length is preferably 22 mm or more and 24 mm or less and still more preferably about 23 mm. The aerosol source 11 is wrapped with the wrapping paper 12 into a cylindrical shape as follows. For example, one edge of the wrapping paper 12 and the opposite edge of the wrapping paper 12 are brought to overlap by about 2 mm in the circumferential direction and bonded together to form a circular paper tube, and the aerosol source 11 is filled into the tubule shape. The size of the rectangular wrapping paper 12 can be determined according to the size of the substrate 10.

The wrapping paper 12 may contain a filler in addition to the pulp. The content of the filler with respect to the total mass of the wrapping paper 12 is 10% by mass or more and less than 60% by mass and preferably 15% by mass or more and 45% by mass or less.

In the wrapping paper 12, the content of the filler is preferably 15% by mass or more and 45% by mass or less when the basis weight is within the preferred range (25 gsm or more and 45 gsm or less).

When the basis weight is 25 gsm or more and 35 gsm or less, the content of the filler is preferably 15% by mass or more and 45% by mass or less. When the basis weight is 35 gsm or more and 45 gsm or less, the content of the filler is preferably 25% by mass or more and 45% by mass or less.

The filler used may be calcium carbonate, titanium dioxide, kaolin, etc. From the viewpoint of flavor and of increasing the whiteness, it is preferable to use calcium carbonate.

Various auxiliary agents other than the base sheet and the filler may be added to the wrapping paper 12. For example, to improve the water resistance, a water resistance improver may be added. Examples of the water resistance improver include a wet strength agent (WS agent) and a sizing agent. Examples of the wet strength agent include urea-formaldehyde resins, melamine formaldehyde resins, and polyamide epichlorohydrin (PAE). Examples of the sizing agent include rosin soap, alkyl ketene dimers (AKDs), alkenyl succinic anhydrides (ASAs), and highly saponified polyvinyl alcohols having a saponification degree of 90% or more.

A strength agent may be added as an auxiliary agent. Examples of the strength agent include polyacrylamide, cationic starch, oxidized starch, CMC, polyamide epichlorohydrin resins, and polyvinyl alcohol. In particular, it is known that the use of a trace amount of oxidized starch improves air permeability (Japanese Unexamined Patent Application Publication No. 2017-218699).

A coating agent may be added to at least one of the front and back surfaces of the wrapping paper 12. No particular limitation is imposed on the coating agent, but it is preferable to use a coating agent that can form a film on the surface of the paper to thereby reduce the liquid permeability of the paper. Examples of the coating agent include alginic acid and salts thereof (such as a sodium salt), polysaccharides such as pectin, cellulose derivatives such as ethyl cellulose, methyl cellulose, carboxymethyl cellulose, and nitrocellulose, and starch and derivatives thereof (e.g., ether derivatives such as carboxymethyl starch, hydroxyalkyl starch, and cationic starch and ester derivatives such as starch acetate, starch phosphate, and starch octenylsuccinate).

[Cooling section 20]

The cooling section 20 is disposed adjacent to the substrate 10 and to the filter section 30 and includes a shaping paper 21 shaped into a tubular shape having a transverse cross section with a hollow (cavity).

The dimension of the cooling section 20 in the centerline direction is 15 mm or more. The dimension of the cooling section 20 in the centerline direction is 35 mm or less, preferably 30 mm or less, and more preferably 25 mm or less. When the dimension of the cooling section 20 in the centerline direction is equal to or more than the above lower limit, a sufficient cooling effect can be achieved, and a good flavor can be obtained. When the dimension of the cooling section 20 in the centerline direction is equal to or less than the above upper limit, the loss of the generated vapor and aerosol due to adhesion to the shaping paper 21 can be reduced.

The shaping paper 21 has a cylindrical shape, and its inside diameter can be appropriately changed according to the size of the product and is preferably substantially the same as the outside diameter of the aerosol source 11 of the substrate 10. In other words, it is preferable that the inside diameter of the shaping paper 21 is substantially the same as the inside diameter of the wrapping paper 12. When the inside diameter of the shaping paper 21 is substantially the same as the inside diameter of the wrapping paper 12, a sufficient passage can be provided for the generated vapor and aerosol, and the loss due to adhesion to the shaping paper 21 can be reduced.

No particular limitation is imposed on the thickness of the shaping paper 21. The thickness may be, for example, 5 $\mu$m or more and 500 $\mu$m or less and may be 10 $\mu$m or more and 250 $\mu$m or less.

No particular limitation is imposed on the material of the shaping paper 21. The material may contain pulp as a main component or may contain polyethylene, polypropylene, polyvinyl chloride, polyethylene terephthalate, polylactic acid, cellulose acetate, or aluminum foil as a main component. Any combination of these materials may also be used.

The shaping paper 21 may be creped to form channels in order to increase the surface area on the inner side, and then the creped paper may be pleated, gathered, and folded to form a thin material sheet.

The cooling section 20 has a plurality of perforations V (which are referred to also as a "ventilation filter (Vf)" in this technical field) arranged concentrically in the circumferential direction. The perforations V are holes passing through the shaping paper 21. The holes have a circular shape. However, the perforations V may have a shape other the circular shape such as a polygonal, rounded polygonal, or elliptical shape. The presence of the perforations V allows air to flow from the outside to the inside of the shaping paper 21 in the cooling section 20 during inhalation, and the temperature of the vapor and air flowing from the substrate 10 can thereby be reduced.

The perforations V are provided such that the ratio of air flowing through the perforations V when an automatic smoking machine is used for inhalation at 17.5 mL/second is 10% by volume or more and 90% by volume or less. The "ratio of air flow" is the volume ratio of the air flowing through the perforations V when the ratio of the air inhaled through a mouthpiece end is set to 100% by volume. The ratio of air flow is preferably 50% by volume or more and 80% by volume or less and more preferably 55% by volume or more and 75% by volume or less. The above ratio of air flow can be achieved, for example, as follows. The number of perforations V in one perforation group is selected from the range of 5 to 50, and the diameter of the perforations V is selected from the range of 0.1 to 0.5 mm. Then the number of perforations V and their diameter are appropriately combined.

The ratio of air flow can be measured using a roll quality measuring device (SODIMAX D74/SODIM manufactured by S.A.S) by a method according to ISO 9512.

The region in which the perforations V are present will be described later.

[Filter section 30]

The filter section 30 includes a first filter 31 connected to the second side of the cooling section 20, a second filter 32 located on the second side of the first filter 31, and a plug wrap paper 33 that is wrapped around the outer side of the first filter 31 and the second filter 32 to connect the first filter 31 and the second filter 32 together. The first filter 31 has a hollow transverse cross section, and the second filter 32 has a solid transverse cross section. The second-side end of the cooling section 20 and the first-side end of the filter section 30 are integrally wrapped with the tipping paper 40, and the filter section 30 is thereby connected (joined) to the cooling section 20.

The transverse cross sections of the outer circumferential portions of the first filter 31 and the second filter 32 of the filter section 30 each have a substantially circular shape, and the diameter of the circle can be appropriately changed according to the size of the product but is generally 4.0 mm or more and 9.0 mm or less, preferably 4.5 mm or more and 8.5 mm or less, and still more preferably 5.0 mm or more and 8.0 mm or less. When the transverse cross section is not circular, the diameter is determined as follows. The cross section is assumed to be a circle with the same area as the cross section, and the diameter of the circle is used.

The circumferential length of the transverse cross section of each of the outer circumferential portions of the first filter 31 and the second filter 32 can be appropriately changed according to the size of the product but is generally 14.0 mm or more and 27.0 mm or less, preferably 15.0 mm or more and 26.0 mm or less, and still more preferably 16.0 mm or more and 25.0 mm or less.

The dimension of the filter section 30 in the centerline direction can be appropriately changed according to the size of the product but is generally 10.0 mm or more and 30.0 mm or less, preferably 12.5 mm or more and 27.5 mm or less, and still more preferably 15.0 mm or more and 25.0 mm or less. The shapes and dimensions of the first filter 31 and the second filter 32 can be appropriately adjusted such that the shape and dimensions of the filter section 30 fall within the above ranges.

No particular limitation is imposed on the air-flow resistance per 120 mm of the dimension of the filter section 30 in the centerline direction. The air-flow resistance is generally 40 minH$_2$O or more and 300 mmH$_2$O or less, preferably 70 mmH$_2$O or more and 280 mmH$_2$O or less, and more preferably 90 mmH$_2$O or more and 260 mmH$_2$O or less.

The air-flow resistance is measured, for example, using a filter air-flow resistance measurement device manufactured by Cerulean according to an ISO standard method (ISO 6565). The air-flow resistance of the filter section 30 is the difference in pressure between the first side and the second side when air is caused to flow from the first side to the second side at a predetermined flow rate (17.5 cc/min) with no air transmission through the side surface of the filter section 30. The unit of the air-flow resistance is generally mmH$_2$O.

No particular limitation is imposed on the first filter 31 and the second filter 32 so long as they each contain a filter material and have the functions of general filters. Examples of the functions of general filters include the function of controlling the amount of air mixed during inhalation of the aerosol etc., the function of reducing the amount of flavor, and the function of reducing the amounts of nicotine and tar. However, it is unnecessary that the filters have all of these functions. In the non-combustion-heating-type stick 2, the number of components generated is smaller than that in cigarette products, and the filling rate of the aerosol source 11 tends to be small. Therefore, the function of preventing the aerosol source 11 from falling off while the filtering function is reduced is one of the important functions.

The filter material forming the first filter 31 and the second filter 32 is a material prepared by forming a filler such as acetate, charcoal, cellulose fibers, a nonwoven fabric, or pulp paper into a cylindrical shape. A paper filter prepared by packing sheet-shaped pulp paper into a filter shape may also be used.

No particular limitation is imposed on the density of the filter material. The density is generally 0.10 g/cm$^3$ or more and 0.25 g/cm$^3$ or less, preferably 0.11 g/cm$^3$ or more and 0.24 g/cm$^3$ or less, and more preferably 0.12 g/cm$^3$ or more and 0.23 g/cm$^3$ or less.

No particular limitation is imposed on the form of the plug wrap paper 33, and the plug wrap paper 33 may include at least one seam including an adhesive. The adhesive may contain a hot-melt adhesive, and the hot-melt adhesive may contain polyvinyl alcohol. When the filter section 30 includes two or more members, it is preferable that the plug wrap paper wraps the two or more members together.

No particular limitation is imposed on the material of the plug wrap paper 33, and any well-known material can be used. The plug wrap paper 33 may contain a filler such as calcium carbonate.

No particular limitation is imposed on the thickness of the plug wrap paper 33, and the thickness is generally 20 $\mu$m or more and 140 $\mu$m or less, preferably 30 $\mu$m or more and 130 $\mu$m or less, and more preferably 30 $\mu$m or more and 120 $\mu$m or less.

No particular limitation is imposed on the basis weight of the plug wrap paper 33, and the basis weight is generally 20 gsm or more and 100 gsm or less, preferably 22 gsm or more and 95 gsm or less, and more preferably 23 gsm or more and 90 gsm or less.

The plug wrap paper 33 may or may not be coated. From the viewpoint that functions other than strength and structural stiffness can be imparted, it is preferable that the plug wrap paper 33 is coated with a desired material.

[Tipping paper 40]

No particular limitation is imposed on the structure of the tipping paper 40, and the tipping paper 40 can have a general form. For example, the tipping paper 40 may include pulp as a main component. The pulp may be made of wood pulp such as soft wood pulp or hard wood pulp or may be produced using a pulp mixture containing non-wood pulp generally used for wrapping paper for tobacco products such as flax pulp, hemp pulp, sisal pulp, or esparto. One type of pulp may be used alone, and a combination of a plurality of types may be used at any ratio.

The tipping paper 40 may be formed of one sheet of paper or may be formed of a plurality of sheets of paper.

Examples of the type of pulp that can be used include chemical pulp, ground pulp, chemiground pulp, and thermomechanical pulp that are produced, for example, by a kraft cooking method, an acidic/neutral/alkaline sulfite cooking method, or a soda salt cooking method.

The tipping paper 40 may be produced by any of the above methods, or a commercial product may be used.

No particular limitation is imposed on the shape of the tipping paper 40, and the tipping paper 40 may have, for example, a square shape or a rectangular shape.

No particular limitation is imposed on the basis weight of the tipping paper 40, and the basis weight is generally 32 gsm or more and 60 gsm or less, preferably 33 gsm or more and 55 gsm or less, and more preferably 34 gsm or more and 53 gsm or less.

No particular limitation is imposed on the air permeability of the tipping paper 40. The air permeability is generally 0 CORESTA Units or more and 30000 CORESTA Units or less and preferably more than 0 CORESTA Units and 10000 CORESTA Units or less. The air permeability is a value measured according to ISO 2965:2009 and is represented as the flow rate (cm$^3$) of gas passing through an area of 1 cm$^2$ per 1 minute when the difference in pressure between both sides of the paper is 1 kPa. 1 CORESTA Unit (1 C.U.) is cm$^3$/(min·cm$^2$) at 1 kPa.

The tipping paper 40 may contain a filler in addition to the pulp, and examples of the filler include metal carbonates such as calcium carbonate and magnesium carbonate, metal oxides such as titanium oxide, titanium dioxide, and aluminum oxide, metal sulfates such as barium sulfate and calcium sulfate, metal sulfides such as zinc sulfide, quartz, kaolin, talc, diatomaceous earth, and gypsum. In particular, from the viewpoint of increasing whiteness-opacity and increasing heating speed, it is preferable that the tipping paper 40 contains calcium carbonate. One of these fillers may be used alone, or a combination of two or more may be used.

Various auxiliary agents other than the pulp and the filler may be added to the tipping paper 40. For example, to improve the water resistance, a water resistance improver may be contained. Examples of the water resistance improver include a wet strength agent (WS agent) and a sizing agent. Examples of the wet strength agent include urea-formaldehyde resins, melamine formaldehyde resins, and polyamide epichlorohydrin (PAE). Examples of the sizing agent include rosin soap, alkyl ketene dimers (AKDs), alkenyl succinic anhydrides (ASAs), and highly saponified polyvinyl alcohols having a saponification degree of 90% or more.

A coating agent may be added to at least one of the front and back surfaces of the tipping paper 40. No particular limitation is imposed on the coating agent, but it is preferable to use a coating agent that can form a film on the surface of the paper to thereby reduce the liquid permeability of the paper.

Part of the outer surface of the tipping paper 40 may be coated with a lip-release material. The lip-release material means a material that, when the user holds the filter section 30 of the stick 2 in the mouth, allows the contact between the tipping paper 40 and the lips to be easily released with substantially no adhesion. The lip-release material may contain, for example, ethyl cellulose, methyl cellulose, etc. For example, an ethyl cellulose-based ink or a methyl cellulose-based ink may be applied to the outer surface of the tipping paper 40 to coat the outer surface of the tipping paper 40 with the lip-release material.

A plurality of through holes 41 are formed in the tipping paper 40 at positions opposed to the perforations V formed in the cooling section 20. No particular limitation is imposed on the shape etc. of the plurality of through holes 41 so long as they are formed so as not to close the plurality of perforations V formed in the cooling section 20. For example, one through hole 41 may be opposed to one perforation V, or one through hole 41 may be opposed to two or more perforations V. To form the through holes 41 so as to be opposed to the perforations V, for example, the tipping paper 40 with the through holes 41 formed therein is wrapped around the outer side of the shaping paper 21 such that the through holes 41 and the perforations V are opposed to each other. Alternatively, with no through holes 41 and no perforations V formed, the tipping paper 40 is wrapped around the outer side of the shaping paper 21, and then the shaping paper 21 and the tipping paper 40 are pierced simultaneously to form the perforations V and the through holes 41.

(Inhaler device 100)

As shown in Fig. 1, the inhaler device 100 includes a power supply 111 that stores electric power and supplies the electric power to each structural element of the inhaler device 100, a sensor 112 that detects various types of information about the inhaler device 100, and a notifier 113 that notifies the user of the information. The inhaler device 100 further includes a memory 114 that stores various types of information for the operation of the inhaler device 100, a communicator 115 for communicating information between the inhaler device 100 and another device, and a controller 116 that controls the overall operation of the inhaler device 100.

The inhaler device 100 further includes a heater 121 that heats the stick 2, a heat insulator 122 that prevents heat transfer from the heater 121 to the other structural elements of the inhaler device 100, and a holder 130 that holds the stick 2. The user of the inhaler device 100 performs inhalation with the stick 2 held in the holder 130.

The inhaler device 100 further includes a housing 110 that accommodates the power supply 111, the sensor 112, the notifier 113, the memory 114, the communicator 115, the controller 116, the heater 121, the heat insulator 122, and the holder 130. The housing 110 has an opening 110a formed in order to insert the stick 2 into the housing 110. Examples of the material forming the housing 110 include plastics and metal materials such as aluminum. The inhaler device 100 may include an open-close lid (not shown) that is attached to the upper surface of the housing 110 in a slidable manner to open/close the opening 110a.

[Holder 130]

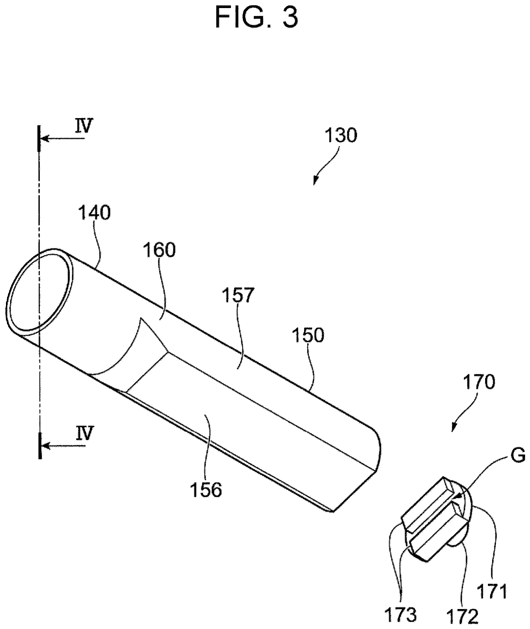

Fig. 3 is an illustration showing an example of a schematic structure of the holder 130.

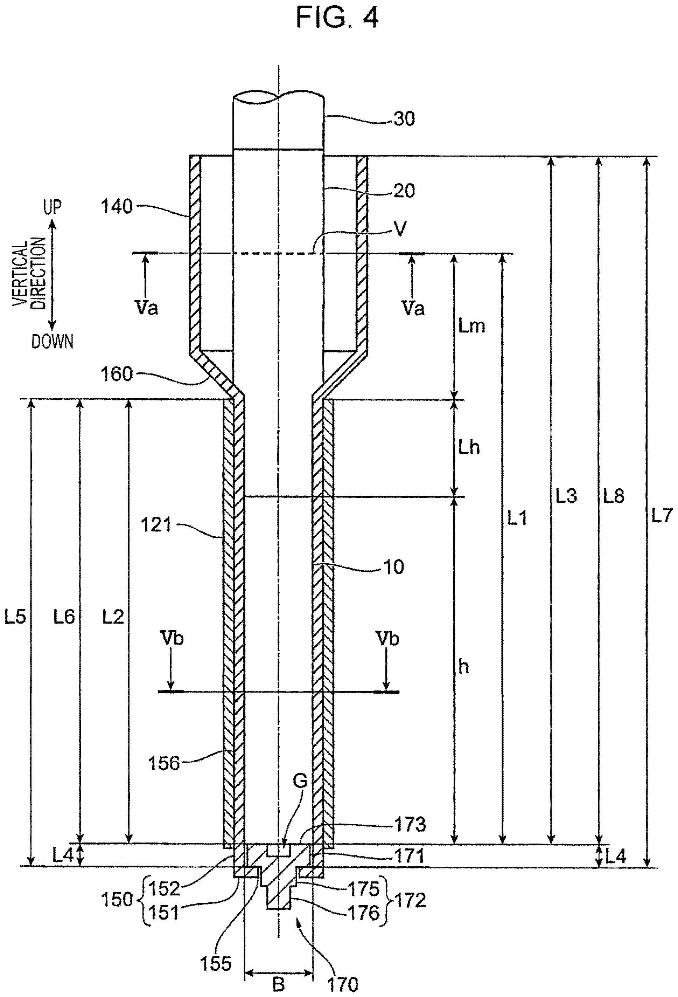

Fig. 4 is an illustration showing an example of a cross section along IV-IV in Fig. 3.

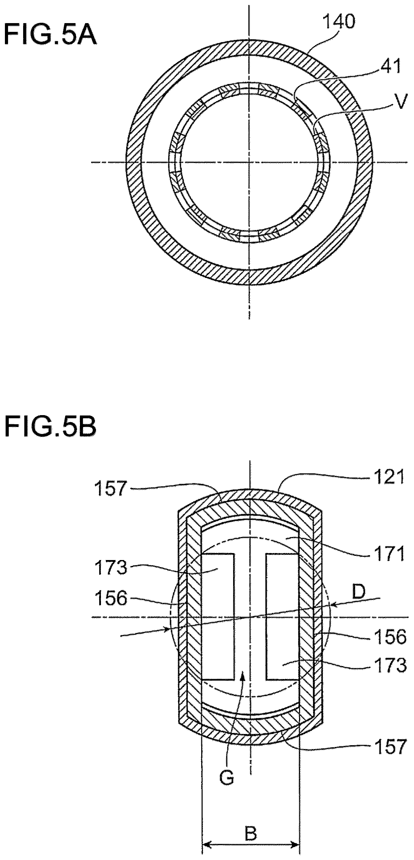

Fig. 5A is an illustration showing an example of a cross section along Va-Va in Fig. 4. Fig. 5B is an illustration showing an example of a cross section along Vb-Vb in Fig. 4.

The holder 130 includes an insertion section 140 located on the side through which the stick 2 is inserted, a compression section 150 in which the stick 2 is compressed in the radial direction, a connection section 160 that connects the insertion section 140 and the compression section 150, and a positioning member 170 that sets the position of the stick 2 in the centerline direction.

The insertion section 140, the compression section 150, and the connection section 160 are tubular portions formed such that their centerlines are aligned with each other. In one example, the insertion section 140, the compression section 150, and the connection section 160 are formed integrally and fixed directly or indirectly to the housing 110 (see Fig. 1). In one example, the material of the insertion section 140, the compression section 150, and the connection section 160 is a metal such as stainless steel. The insertion section 140, the compression section 150, and the connection section 160 may contain a ceramic material. Examples of the ceramic material include alumina, aluminum nitride, and silicon nitride ceramics. These ceramic materials may be stacked together and sintered.

The holder 130 holds the stick 2 inserted through the insertion section 140 with the stick 2 compressed in the compression section 150. With the holder 130 holding the stick 2, the centerline direction of the insertion section 140, the compression section 150, and the connection section 160 coincides with the centerline direction of the stick 2. The holder 130 holds the stick 2 such that the first-side end, with respect to the centerline direction, of the stick 2 is in contact with the positioning member 170 within the housing 110 and that the second-side end, with respect to the centerline direction, of the stick 2 is exposed to the outside of the housing 110. The centerline direction of the stick 2 with the holder 130 holding the stick 2 is referred to as a vertical direction. The side on which the first-side end, with respect to the centerline direction, of the stick 2 is located may be referred to as a lower side, and the side on which the second-side end, with respect to the centerline direction, of the stick 2 is located may be referred to as an upper side.

The insertion section 140 is a cylindrical portion. The inside diameter of the insertion section 140 is larger than the outside diameter of the tipping paper 40 that is disposed on the outermost side of the stick 2. The upper opening of the insertion section 140 is disposed at a position opposed to the opening 110a of the housing 110. For example, the insertion section 140 and the housing 110 are disposed such that, when the opening 110a of the housing 110 is circular, the center of the opening 110a and the center of the insertion section 140 coincide with each other when the opening 110a and the insertion section 140 are viewed in the vertical direction. As shown in Fig. 1, the insertion section 140 and the housing 110 are disposed such that the upper edge of the insertion section 140 and the inner surface of the housing 110 are in contact with each other or that a small gap (e.g., 0.5 mm) is formed therebetween. Therefore, the upper edge of the insertion section 140 is located inside the housing 110 and spaced from of the upper surface of the housing 110 by the sum of the wall thickness of the housing 110 and the gap between the inner surface of the housing 110 and the upper edge of the insertion section 140. For example, when the wall thickness of the housing 110 is 0.5 mm and the gap between the upper edge of the insertion section 140 and the inner surface of the housing 110 is 0.5 mm, the upper edge of the insertion section 140 is present at a position 1 mm inward from the upper surface of the housing 110. From the viewpoint of introducing a larger amount of air from the outside of the housing 110 into the insertion section 140, the closer the upper edge of the insertion section 140 is to the upper surface of the housing 110, the better. It is desirable that the upper edge of the insertion section 140 is present within 1 mm from the upper surface of the housing 110.

The compression section 150 is a closed-bottom tubular portion and has a bottom 151 and a tubular portion 152. The bottom 151 has a through hole 155 formed at its center.

The tubular portion 152 has a pair of opposed flat walls 156 that are disposed so as to be opposed to each other with a gap smaller than the outside diameter D of the substrate 10 of the stick 2 therebetween. The compression section 150 differs from the insertion section 140 in that the pair of opposed walls 156 are provided when the compression section 150 is viewed in the vertical direction. Specifically, the compression section 150 includes a pair of arc-shaped portions 157 having the same shape as the insertion section 140 and the pair of opposed walls 156 disposed on both sides of the pair of arc-shaped portions 157.

The pair of opposed walls 156 are disposed so as to be parallel to the vertical direction, and the gap B between the pair of opposed walls 156 is set to be smaller than the outside diameter D of the substrate 10 of the stick 2. In a transverse cross section of the pair of arc-shaped portions 157, the diameter of an imaginary circle passing through the inner surfaces of the pair of arc-shaped portions 157 is larger than the outside diameter D of the substrate 10 of the stick 2.

The connection section 160 is a portion that is disposed between the lower edge of the insertion section 140 and the upper edge of the compression section 150 and that has a transverse cross section that gradually changes from the transverse cross section of the insertion section 140 to the transverse cross section of the compression section 150 as one goes from the top to the bottom.

The positioning member 170 is a member attached inside the compression section 150. The positioning member 170 has a columnar main portion 171 having an outer surface extending along the bottom 151 of the compression section 150 and the inner surface of the tubular portion 152, a downward protruding portion 172 protruding downward from the lower surface of the main portion 171, and a pair of upward protruding portions 173 protruding upward from the upper surface of the main portion 171.

The downward protruding portion 172 has a first columnar portion 175 having a columnar shape and a second columnar portion 176 having a dimeter smaller than the diameter of the first columnar portion 175. The first columnar portion 175 and the second columnar portion 176 are arranged in the vertical direction such that the first columnar portion 175 is on the upper side and the second columnar portion 176 is on the lower side. The diameter of the first columnar portion 175 is slightly smaller than the diameter of the through hole 155 formed on the bottom 151 of the compression section 150. The positioning member 170 is attached to the compression section 150 with the first columnar portion 175 and the second columnar portion 176 passing through the through hole 155 so as to be exposed to the outside of the compression section 150.

The upward protruding portions 173 each have a rectangular parallelepipedic shape and are arranged such that, when the upward protruding portions 173 are viewed in the vertical direction, the direction along the inner surfaces of the opposed walls 156 of the compression section 150 coincides with the longitudinal direction of the upward protruding portions 173 and that the direction in which the pair of opposed walls 156 face each other coincides with the short-side direction. Specifically, the pair of upward protruding portions 173 are separated by a predetermined gap G in the direction in which the pair of opposed walls 156 face each other. The dimension of each of the upward protruding portions 173 in the longitudinal direction is larger than the gap B between the pair of opposed walls 156, and this can prevent the pair of the upward protruding portions 173 from being attached to the compression section 150 with each of the upward protruding portions 173 in contact with both the opposed walls 156. Moreover, the pair of upward protruding portions 173 have the same vertical dimension.

In the positioning member 170 having the above structure, when the stick 2 is inserted into the compression section 150, the upper surfaces of the pair of upward protruding portions 173 serve as portions in contact with the forward end of the substrate 10 of the stick 2 (the first-side end surface with respect to the centerline direction). In other words, the stick 2 inserted into the compression section 150 is inserted to a prescribed position at which the forward end of the substrate 10 comes into contact with the pair of upward protruding portions 173. Then, with the stick 2 inserted into the compression section 150, the substrate 10 is sandwiched between the pair of opposed walls 156 of the compression section 150 and thereby compressed. With the stick 2 in contact with the pair of upward protruding portions 173, the forward end of the substrate 10 straddles the upper portion of the gap G formed between the pair of upward protruding portions 173.

[Heater 121]

Examples of the heater 121 include thin metal film heaters and film heaters disposed along the outer circumferential surface of the tubular portion 152 of the compression section 150. A thin metal film heater is a flexible planar heat-generating heater using a thin metal film as a heat generator. A film heater includes, for example, a metal used as a heat generator and insulating films disposed on opposite sides of the metal to insulate the metal. Examples of the metal used as the heat generator include stainless steel, and examples of the insulating films include polyimides.

The heater 121 is electrically connected to the power supply 111 via electrical wiring. When electric power is supplied from the power supply 111, the heater 121 generates heat and heats the holder 130. As a result, the substrate 10 of the stick 2 inserted into the holder 130 is heated from the outer circumferential side through the holder 130.

[Heat insulator 122]

The heat insulator 122 is disposed so as to cover at least the outer circumferential surface of the heater 121. For example, the heat insulator 122 is formed from a vacuum heat insulator, an aerogel heat insulator, etc. The vacuum heat insulator is a heat insulator including, for example, glass wool, silica (silicon powder), etc. wrapped with a resin-made film, and the wrapped heat insulator is evacuated to a high-vacuum state, so that the heat conduction by gas is reduced to as close to zero as possible. The heat insulator 122 prevents the heat generated by the operation of the heater 121 from transferring to the other structural elements of the inhaler device 100.

(Region in which perforations V are present)

Next, a description will be given of the region in which the perforations V formed in the cooling section 20 of the stick 2 are present. When a reference is made to the position of each perforation V in the centerline direction, the position means the position of the center of the circular perforation V. When the through holes 41 are formed together with the perforations V by, after the tipping paper 40 is wrapped around the outer side of the shaping paper 21, piercing the shaping paper 21 and the tipping paper 40 simultaneously, the perforations V and the through holes 41 are present in the same region.

Preferably, the perforations V are formed at positions at least 7 mm from the boundary between the cooling section 20 and the filter section 30 in the direction toward the cooling section 20 (the first side). This is not only because the cooling ability can be improved but also because a product generated when the substrate 10 is heated can be prevented from staying in the cooling section 20 to thereby increase the amount of the aerosol. Moreover, when the substrate 10 is heated, vapor is generated with the aerosol serving as condensation nuclei. Then, when the vapor comes into contact with air from the outside before the vapor adheres to the inner surface of the shaping paper 21, the temperature of the vapor decreases, and the vapor is liquefied, so that the generation of the aerosol is facilitated.

Preferably, the perforations V are formed at positions within 12 mm from the boundary between the cooling section 20 and the filter section 30 in the direction toward the cooling section 20 (the first side). In other words, it is preferable that the perforations V are present in a region spaced a predetermined distance from the boundary between the cooling section 20 and the substrate 10 in the direction toward the cooling section 20 (the second side). This is because the air heated when the substrate 10 is heated is prevented from flowing through the perforations V into the cooling section 20.

The positions of the perforations V will next be considered with respect to the boundary between the cooling section 20 and the substrate 10. When the dimension of the cooling section 20 in the centerline direction is 20 mm, it is preferable that the perforations V are formed at positions 8 mm or more and 13 mm or less from the boundary between the cooling section 20 and the substrate 10 in the direction toward the cooling section 20 (the second side).

When a plurality of perforations V disposed concentrically are treated as one perforation group, the number of perforation groups may be one or may be two or more. When two or more perforation groups are present, it is preferable that these perforation groups are formed at the positions described above.

[Positional relation between perforations V and inhaler device 100]

In the inhalation system 1, the stick 2 and the inhaler device 100 are configured such that, with the holder 130 holding the stick 2, the perforations V are present within the housing 110 and within the insertion section 140 or the connection section 160. This is because of the following reason. If the perforations V formed in the outer circumferential portion of the cooling section 20 are compressed in the tubular portion 152 of the compression section 150, the amount of air flowing through the perforations V into the cooling section 20 is reduced. Therefore, the perforations V are formed such that no perforations are present in the tubular portion 152 of the compression section 150. The phrase "the perforations V are present within the insertion section 140 or the connection section 160" means that the vertical region in which the perforations V are present overlaps the vertical region in which the insertion section 140 or the connection section 160 is provided.

More specifically, the distance L1 from the first-side end surface (forward end surface), with respect to centerline direction, of the stick 2 to the perforations V is set so as to be larger than the distance L2 from the upper surfaces of the pair of upward protruding portions 173 of the positioning member 170 to the lower edge of the connection section 160 (in other words, the upper edge of the tubular portion 152 of the compression section 150) and so as to be smaller than the distance L3 from the upper surfaces of the pair of upward protruding portions 173 to the upper edge of the insertion section 140.

The lower surface of the main portion 171 of the positioning member 170 is in contact with the upper surface of the bottom 151 of the compression section 150. Therefore, let the distance L1 from the first-side end surface, with respect to the centerline direction, of the stick 2 to the perforations V be set in another way. Specifically, the sum of the vertical dimension of the main portion 171 and the vertical dimension of the upward protruding portions 173 is denoted by L4. Then the distance L1 is set so as to be larger than the distance L6 obtained by subtracting the distance L4 from the distance L5 from the lower edge of the connection section 160 to the upper surface of the bottom 151 of the compression section 150 and so as to be smaller than the distance L8 obtained by subtracting the distance L4 from the distance L7 from the upper edge of the insertion section 140 to the upper surface of the bottom 151 of the compression section 150.

The heater 121 is disposed so as to extend to the upper edge of the tubular portion 152 of the compression section 150. From the viewpoint of increasing the temperature of the substrate 10 heated by the heater 121 to thereby increase the amount of the aerosol generated, it is preferable that the heater 121 heats the substrate 10 of the stick 2 and also heats a portion of the cooling section 20 that is located on the substrate 10 side. For example, it is preferable to heat a region within a predetermined heating distance Lh from the boundary between the cooling section 20 and the substrate 10 in the direction toward the cooling section 20. Therefore, it is preferable that the distance L2 from the upper surfaces of the pair of upward protruding portions 173 of the positioning member 170 to the upper edge of the tubular portion 152 of the compression section 150 (in other words, the lower edge of the connection section 160) is equal to a distance obtained by adding the heating distance Lh to the dimension h of the substrate 10 of the stick 2 in the centerline direction. The heating distance Lh is, for example, 5 mm.

Preferably, no perforations V are present near the heater 121. This is because, if warm air heated by the heater 121 flows through the perforations V into the cooling section 20, the generation of the aerosol is not easily facilitated. Therefore, it is preferable that the vertical distance from the upper edge of the tubular portion 152 of the compression section 150 to the perforations V is equal to or more than a predetermined lower limit distance Lm. The lower limit distance Lm is, for example, 3 mm.

Therefore, in consideration of the positional relation with the inhaler device 100, it is preferable that the perforations V are formed at positions spaced equal to or more than (the heating distance Lh + the lower limit distance Lm) from the boundary between the cooling section 20 and the substrate 10 in the direction toward the cooling section 20. For example, when the heating distance Lh is 5 mm and the lower limit distance Lm is 3 mm, it is preferable that the perforations V are formed at positions at least 8 mm from the boundary between cooling section 20 and the substrate 10 in the direction toward the cooling section 20.

For example, when the vertical dimension of the connection section 160 is less than the lower limit distance Lm, the perforations V are present within the insertion section 140.

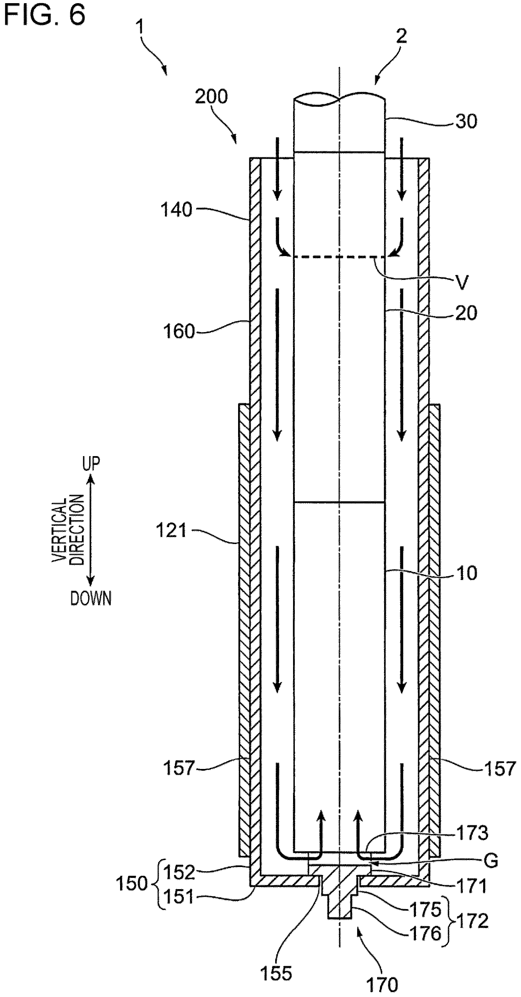

Fig. 6 is an illustration showing an example of the flow of air in the inhalation system 1 during inhalation.

When the inhaler device 100 is used for inhalation, the stick 2 is inserted to the prescribed position at which the forward end of the substrate 10 is in contact with the pair of upward protruding portions 173 of the holder 130. When the stick 2 is inserted to the prescribed position, the forward end of the substrate 10 straddles the upper portion of the gap G formed between the pair of upward protruding portions 173. A gap is present between the inner surface of the pair of arc-shaped portions 157 of the holder 130 and the outer surface of the stick 2. Therefore, when the user performs inhalation, air present outside the housing 110 is introduced into the substrate 10 from the forward end of the substrate 10 through the gap between the inner surface of the insertion section 140 of the holder 130 and the outer surface of the stick 2, the gap between the inner surface of the connection section 160 and the outer surface of the stick 2, the gap between the inner surface of the pair of arc-shaped portions 157 of the compression section 150 and the outer surface of the stick 2, and the gap G between the pair of upward protruding portions 173. The air introduced from the forward end of the substrate 10 into the substrate 10 and the vapor generated by heating the substrate 10 are mixed and introduced into the cooling section 20.

Part of the air introduced from the outside of the housing 110 into the gap between the inner surface of the insertion section 140 of the holder 130 and the outer surface of the stick 2 flows through the perforations V into the cooling section 20. In this manner, the vapor generated by heating the substrate 10 comes into contact with the air flowing through the perforations V into the cooling section 20 and is thereby reduced in temperature, so that the generation of the aerosol is facilitated. Moreover, the air flowing through the perforations V into the cooling section 20 prevents a gas mixture of the air introduced from the forward end of the substrate 10 into the substrate 10 and the vapor generated by heating the substrate 10 from staying in the cooling section 20, so that the amount of the aerosol delivered is increased.

In the inhalation system 1, with the holder 130 holding the stick 2, the perforations V are present within the housing 110 and within the insertion section 140 or the connection section 160. In this case, a gap is present between the inlets of all the plurality of perforations V arranged in the circumferential direction and the inner surface of the insertion section 140 or the connection section 160. Therefore, as compared to the case where the perforations V are assumed to be present within the compression section 150, the amount of air flowing through the perforations V is larger, so that a larger amount of the aerosol can be supplied to the oral cavity of the user

When the centerline direction dimension of a portion of the stick 2 that is exposed from the housing 110 is large, the stick 2 may bend if the user touches the stick 2 with the hand during inhalation or the stick 2 comes into contact with an object such as a desk different from the inhaler device 100. Therefore, the centerline direction dimension of the portion of the stick 2 that is exposed from the upper surface of the housing 110 is preferably 25 mm or less. Since the user holds the portion of the stick 2 that is exposed from the upper surface of the housing 110 in the mouth for inhalation, the centerline direction dimension of the portion of the stick 2 that is exposed from the upper surface of the housing 110 is preferably 10 mm or more.

It is preferable to set the dimensions of the filter section 30, the insertion section 140, and the connection section 160 such that, with the holder 130 holding the stick 2, the perforations V are present within the insertion section 140 or the connection section 160 even when the centerline direction dimension of the portion of the stick 2 that is exposed from the upper surface of the housing 110 is any value in the range of 10 mm or more and 25 mm or less.

For example, when the centerline direction dimension of the portion of the stick 2 that is exposed from the upper surface of the housing 110 is 25 mm, the centerline direction dimension of the filter section 30 is set to 20 mm, and the perforations V are formed at positions 10 mm from the boundary between the cooling section 20 and the filter section 30 in the direction toward the cooling section 20. In this case, the perforations V are present at positions 5 mm inward from the opening 110a of the housing 110. Therefore, when the centerline direction dimension of the insertion section 140 is set to 5 mm or more, the perforations V can be present within the insertion section 140.

As described above, the stick 2 includes the substrate 10 including the aerosol source, the tubular cooling section 20 in which the vapor generated by heating the substrate 10 is cooled to generate the aerosol, and the filter section 30 through which the aerosol passes. The perforations V that allow air to flow from the outside to the inside of the cooling section 20 are formed in the cooling section 20 at positions at least 7 mm from the boundary between the cooling section 20 and the filter section 30 or within 13 mm from the boundary between the cooling section 20 and the substrate 10. In this stick 2, the vapor generated by heating the substrate 10 is unlikely to stay inside the cooling section 20, and the temperature of the vapor decreases before it adheres to the inner surface of the shaping paper 21. Therefore, the generation of the aerosol is facilitated, and the amount of the aerosol that can be supplied to the oral cavity of the user increases.

The perforations V are formed at positions at least 8 mm from the boundary between the cooling section 20 and the substrate 10. In this case, the air heated when the substrate 10 is heated is prevented from flowing through the perforations V into the cooling section 20. Therefore, the amount of the aerosol that can be supplied to the oral cavity of the user is larger than that when the perforations V are formed at positions less than 8 mm from the boundary between the cooling section 20 and the substrate 10.

Similarly, it is preferable that the perforations V are formed at positions within 12 mm from the boundary between the cooling section 20 and the filter section 30. In this case, the perforations V are unlikely to be close to a portion for heating the substrate 10. Therefore, the amount of the aerosol that can be supplied to the oral cavity of the user is larger than that when the perforations V are formed at positions more than 12 mm from the boundary between the cooling section 20 and the filter section 30.

The inhalation system 1 includes: the stick 2 including the substrate 10 including the aerosol source, the tubular cooling section 20 in which the vapor generated by heating the substrate 10 is cooled to generate the aerosol, and the filter section 30 through which the aerosol passes; and the inhaler device 100 including the holder 130 that holds the stick 2, the heater 121 that heats the substrate 10, and the housing 110 that accommodates the holder 130 and the heater 121. In the inhaler device 100, at least the substrate 10 of the stick 2 inserted into the housing 110 through the opening 110a formed in the housing 110 is held by the holder 130, and at least part of the filter section 30 is exposed to the outside of the housing 110. The perforations V that allow air to flow from the outside to the inside of the stick 2 are formed in the cooling section 20 of the stick 2 at positions that are within the housing 110 with the stick 2 held by the holder 130. In this case, the perforations V are easily present at positions that are spaced from the boundary between the cooling section 20 and the filter section 30 so as to be closer to the substrate 10, so that air flows through the perforations V into the cooling section 20 from the positions close to the substrate 10. Therefore, the vapor generated by heating the substrate 10 is unlikely to stay inside the cooling section 20, and the temperature of the vapor decreases before it adheres to the inner surface of the shaping paper 21. In this manner, the generation of the aerosol is facilitated, and the amount of the aerosol that can be supplied to the oral cavity of the user increases.

With the holder 130 holding the stick 2, a gap is formed between the inner surface of the holder 130 and the outer surface of the portion of the cooling section 20 of the stick 2 in which the perforations V are formed. In this case, air can easily flow through the perforations V into the cooling section 20.

The holder 130 includes the insertion section 140 disposed on the opening 110a side and the compression section 150 that is disposed on the opposite side from the opening 110a with respect to the insertion section 140 and that compresses the stick 2 in a direction intersecting the insertion direction. The insertion section 140 has a cylindrical shape having an inside diameter larger than the outside diameter of the outer surface of the stick 2, and the perforations V are located within the insertion section 140 with the holder 130 holding the stick 2. In this case, with the holder 130 holding the stick 2, a gap is accurately formed between the inner surface of the insertion section 140 and the outer surface of the portion of the cooling section 20 of the stick 2 in which the perforations V are formed. Therefore, air can easily flow through the perforations V into the cooling section 20.

The perforations V may be present within the connection section 160 with the holder 130 holding the stick 2. With the holder 130 holding the stick 2, a gap is also formed between the inner surface of the connection section 160 and the outer surface of the portion of the cooling section 20 of the stick 2 in which the perforations V are formed. Therefore, air can easily flow through the perforations V into the cooling section 20.

<Second embodiment>

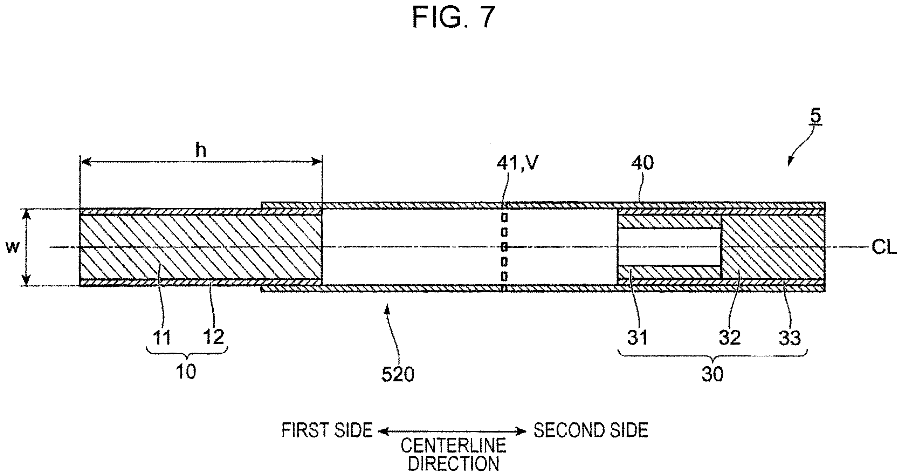

Fig. 7 is an illustration showing a vertical cross section of a stick 5 according to a second embodiment.

The stick 5 according to the second embodiment differs from the stick 2 described above in that the stick 5 includes a cooling section 520 corresponding to the cooling section 20. Hereinafter, differences from the first embodiment will be described. The same parts as those in the first embodiment are denoted by the same symbols, and their description is omitted.

The cooling section 520 differs from that in the stick 2 in that a member corresponding to the shaping paper 21 is not included. The cooling section 520 is a space formed inside the stick 5 and is a tubular space surrounded by the substrate 10, the filter section 30, and the tipping paper 40. In the stick 5 according to the second embodiment, the through holes 41 formed in the tipping paper 40 function as perforations V that allow air to flow from the outside to the inside of the cooling section 520.

In the stick 5 according to the second embodiment having the structure described above also, the centerline direction positions of the perforations V formed are similar to the centerline direction positions of the perforations V in the stick 2 according to the first embodiment. Therefore, the same effects as those described in the first embodiment can be obtained.

Reference Signs List

1 inhalation system, 2, 5 non-combustion-heating-type stick, 10 substrate, 20 cooling section, 30 filter section, 40 tipping paper, 110 housing, 110a opening, 121 heater, 130 holder, 140 insertion section, 150 compression section, V perforation

## Claims

1.  A non-combustion-heating-type stick comprising:

    a substrate including an aerosol source;
    a tubular cooling section for cooling vapor generated by heating the substrate to thereby generate an aerosol; and
    a filter section through which the aerosol passes, wherein a perforation that allows air to flow from an outside of the cooling section into an inside of the cooling section is formed in the cooling section at a position at least 7 mm from a boundary between the cooling section and the filter section or at a position within 13 mm from a boundary between the cooling section and the substrate.

2.  The non-combustion-heating-type stick according to claim 1, wherein the perforation is formed at a position at least 8 mm from the boundary between the cooling section and the substrate.

3.  The non-combustion-heating-type stick according to claim 1, wherein the perforation is formed at a position within 12 mm from the boundary between the cooling section and the filter section.

4.  An inhalation system comprising:

    a non-combustion-heating-type stick including a substrate including an aerosol source, a tubular cooling section for cooling vapor generated by heating the substrate to thereby generate an aerosol, and a filter section through which the aerosol passes; and
    an inhaler device including a holder that holds the non-combustion-heating-type stick, a heater that heats the substrate, and a housing that accommodates the holder and the heater, wherein, in the inhaler device, the holder holds at least the substrate of the non-combustion-heating-type stick inserted into the housing through an opening formed in the housing with at least part of the filter section exposed to an outside of the housing, and
    wherein, in the cooling section of the non-combustion-heating-type stick, a perforation that allows air to flow from an outside of the cooling section into an inside of the cooling section is formed at a position that, with the non-combustion-heating-type stick held by the holder, is located within the housing.

5. The inhalation system according to claim 4, wherein, in the holder with the non-combustion-heating-type stick held thereby, a gap is formed between an inner surface of the holder and an outer surface of a portion of the cooling section of the non-combustion-heating-type stick, which the portion has the perforation formed therein.

6. The inhalation system according to claim 5, wherein the holder includes an insertion section disposed on an opening side and a compression section that is disposed on an opposite side from the opening with respect to the insertion section and that compresses the non-combustion-heating-type stick in a direction intersecting an insertion direction, wherein the insertion section has a cylindrical shape having an inside diameter larger than an outside diameter of the outer surface of the non-combustion-heating-type stick, and

wherein, with the holder holding the non-combustion-heating-type stick, the perforation is located within the insertion section.

7. The inhalation system according to any one of claims 4 to 6, wherein the perforation is formed at a position at least 7 mm from a boundary between the cooling section and the filter section or at a position within 13 mm from a boundary between the cooling section and the substrate.

## FIG. 1

EP 4 445 755 A1

# FIG. 2

FIRST SIDE ←————→ SECOND SIDE
CENTERLINE
DIRECTION

# FIG. 3

# FIG. 4

FIG.5A

FIG.5B

## FIG. 6

# FIG. 7

FIRST SIDE ←————→ SECOND SIDE

CENTERLINE
DIRECTION

EP 4 445 755 A1

**INTERNATIONAL SEARCH REPORT**

International application No.

**PCT/JP2021/045032**

**A. CLASSIFICATION OF SUBJECT MATTER**

*A24D 1/20*(2020.01)i; *A24F 40/20*(2020.01)i
FI:  A24D1/20; A24F40/20

According to International Patent Classification (IPC) or to both national classification and IPC

**B. FIELDS SEARCHED**

Minimum documentation searched (classification system followed by classification symbols)

A24D1/20; A24F40/20

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2021-503880 A (NICOVENTURES TRADING LTD) 15 February 2021 (2021-02-15) paragraphs [0081], [0089], [0091], [0096], [0099], fig. 5, 8, 9 | 1-5, 7 |
| A | | 6 |
| A | WO 2020/100927 A1 (JAPAN TOBACCO INC) 22 May 2020 (2020-05-22) | 1-7 |
| A | JP 2021-514644 A (KT & G CORP) 17 June 2021 (2021-06-17) | 1-7 |

☐ Further documents are listed in the continuation of Box C.    ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **24 January 2022** | **08 February 2022** |

| Name and mailing address of the ISA/JP | Authorized officer |
|---|---|
| **Japan Patent Office (ISA/JP)** 3-4-3 Kasumigaseki, Chiyoda-ku, Tokyo 100-8915 Japan | |
| | Telephone No. |

Form PCT/ISA/210 (second sheet) (January 2015)

| INTERNATIONAL SEARCH REPORT | | International application No. |
|---|---|---|
| Information on patent family members | | **PCT/JP2021/045032** |

| Patent document cited in search report | | | Publication date (day/month/year) | Patent family member(s) | | | Publication date (day/month/year) |
|---|---|---|---|---|---|---|---|
| JP | 2021-503880 | A | 15 February 2021 | US | 2021/0100283 | A1 | |
| | | | | paragraphs [0090], [0098], [0100], [0105], [0108], fig. 5, 8, 9 | | | |
| | | | | WO | 2019/105750 | A1 | |
| | | | | EP | 3716801 | A1 | |
| | | | | CN | 111629617 | A | |
| WO | 2020/100927 | A1 | 22 May 2020 | CN | 113038846 | A | |
| JP | 2021-514644 | A | 17 June 2021 | US | 2021/0000180 | A1 | |
| | | | | WO | 2020/105944 | A1 | |
| | | | | EP | 3818879 | A1 | |
| | | | | CN | 111757677 | A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019518450 W
- WO 2020100927 A
- JP 2004510422 W
- WO 2014104078 A
- JP 2017218699 A

**Non-patent literature cited in the description**

- Tobacco no Jiten (Dictionary of Tobacco). Tobacco Academic Studies Center, 31 March 2009